# EUROPEAN PATENT APPLICATION

(11) **EP 3 251 697 A1**
(43) Date of publication of application: **06.12.2017**
(21) Application number: 16743356.4
(22) Date of filing: 26.01.2016
(51) Int. Cl.: A61K 47/50, A61K 9/08, A61K 31/192, A61K 31/405, A61K 31/616, A61K 47/18

(54) **METHOD FOR CONTROLLING SKIN PENETRATION**

(30) Priority: 27.01.2015 JP 2015013329
(71) Applicant: AJINOMOTO CO., INC., Chuo-ku Tokyo 104-8315 (JP)
(72) Inventor: FURUKAWA, Shinya, Kawasaki-shi Kanagawa 210-8681 (JP); HATTORI, Gaku, Kawasaki-shi Kanagawa 210-8681 (JP)
(74) Representative: Dunne, Paul David
(86) International application number: PCT/JP2016/052171
(87) International publication number: WO 2016/121753

(57) **Abstract**

An object of the present invention is to provide a method for controlling skin permeability of an anionic compound and having superior safety.

An ionic liquid formed by an anionic compound and an amino acid ester is used.

## Description

### [Technical Field]

The present invention relates to a method for controlling skin permeation, and further to a skin external preparation utilizing the method for controlling skin permeation, and a production method of the skin external preparation.

### [Background Art]

Ionic liquid refers to an ordinary temperature-molten salt constituted of anion and cation, and is known as a new solvent next to water and organic solvents. Ever since it has been reported to have superior property as a carrier such as bioactive substance (e.g., efficacy component) and the like, a wide variety of studies are ongoing in various fields.

In recent years, it has been reported that when an ionic liquid is applied to the skin, it exhibits markedly high skin permeability, and various studies have been made on application to skin penetrating agents making use of such property. For example, attempts have been made to improve skin permeability of a bioactive substance by dispersing or dissolving the bioactive substance in an ionic liquid and applying same to the skin. However, according to such method, the bioactive substance does not penetrate the skin in complete association with the ionic liquid and the arrival rate of the bioactive substance at the corium was low.

On the other hand, a method of improving skin permeability of a bioactive substance by converting the bioactive substance itself into an ionic liquid and applying same to the skin has also been reported (e.g., patent documents 1 - 4 etc.). As compared to a method including dispersing or dissolving the bioactive substance in an ionic liquid, the method shows a high arrival rate of the bioactive substance at the corium, since the content of the efficacy component per unit volume in the ionic liquid is high and the bioactive substance can penetrate the skin in complete association with the ionic liquid.

### [Document List]

### [Patent documents]

patent document 1: WO 2010/016219
patent document 2: WO 2009/075094
patent document 3: WO 2009/060629
patent document 4: WO 2008/093686

### [SUMMARY OF THE INVENTION]

### [Problems to be Solved by the Invention]

The present inventors performed an evaluation test of the skin permeation rate of an ionic liquid constituted of amino acid anion and tetrabutylphosphonium, and confirmed that the ionic liquid shows a remarkable skin permeation rate. However, an ionic liquid containing an organophosphorus compound such as tetrabutylphosphonium and the like as a constituent component is feared to have a safety problem.

An object of the present invention is to provide a method for controlling skin permeation of the active ingredient by using an ionic liquid more superior in the safety to the living body. Furthermore, an object of the present invention is to provide a skin external preparation showing skin permeability controlled by the method, and a method of producing the skin external preparation.

### [Means of Solving the Problems]

The present inventors have conducted intensive studies in an attempt to solve the above-mentioned problems and found that an ionic liquid having amino acid as the basic skeleton and superior in safety can be obtained by converting an anionic compound into an ionic liquid by using amino acid ester, and skin permeability of the anionic compound can be controlled by applying the ionic liquid to the skin.

The present inventors have also found that, for example, an anionic compound can be retained at a high concentration in the skin by applying the ionic liquid to the skin.

The present inventors have conducted further studies based on the finding, and completed the present invention. Accordingly, the present invention provides the following.
[1] A skin external preparation comprising an ionic liquid formed by an anionic compound and an amino acid ester.
[2] The skin external preparation of [1], wherein the amino acid ester is a lower alcohol ester of amino acid.
[3] The skin external preparation of [2], wherein the lower alcohol is at least one kind selected from the group consisting of methanol, ethanol, 1-propanol and 2-propanol.
[4] The skin external preparation of any one of [1] - [3], wherein the anionic compound has at least one kind of functional group selected from the group consisting of a carboxy group, a sulfo group, a phosphoric acid group and a phosphono group.
[5] The skin external preparation of any one of [1] - [4], wherein the anionic compound is hardly soluble in water.
[6] The skin external preparation of any one of [1] - [5], wherein the anionic compound has a molecular weight of 30 - 5000.
[7] The skin external preparation of any one of [1] - [6], wherein the anionic compound is a bioactive substance.
[8] The skin external preparation of any one of [1] - [7], wherein the anionic compound is solid at 25°C.
[9] A method for controlling skin permeation of an anionic compound, comprising converting the anionic compound into an ionic liquid with an amino acid ester.
[10] The method for controlling the skin permeation of [9], wherein the amino acid ester is a lower alcohol ester of amino acid.
[11] The method for controlling the skin permeation of [10], wherein the lower alcohol is at least one kind selected from the group consisting of methanol, ethanol, 1-propanol and 2-propanol.
[12] The method for controlling the skin permeation of any one of [9] - [11], wherein the anionic compound has at least one kind of functional group selected from the group consisting of a carboxy group, a sulfo group, a phosphoric acid group and a phosphono group.
[13] The method for controlling the skin permeation of any one of [9] - [12], wherein the anionic compound is hardly soluble in water.
[14] The method for controlling the skin permeation of any one of [9] - [13], wherein the anionic compound has a molecular weight of 30 - 5000.
[15] The method for controlling the skin permeation of any one of [9] - [14], wherein the anionic compound is a bioactive substance.
[16] The method for controlling the skin permeation of any one of [9] - [15], wherein the anionic compound is solid at 25°C.
[17] A method of producing a skin external preparation comprising an ionic liquid, comprising forming the ionic liquid from an anionic compound and an amino acid ester.
[18] The production method of [17], wherein the amino acid ester is a lower alcohol ester of amino acid.
[19] The production method of [18], wherein the lower alcohol is at least one kind selected from the group consisting of methanol, ethanol, 1-propanol and 2-propanol.
[20] The production method of any one of [17] - [19], wherein the anionic compound has at least one kind of functional group selected from the group consisting of a carboxy group, a sulfo group, a phosphoric acid group and a phosphono group.
[21] The production method of any one of [17] - [20], wherein the anionic compound is hardly soluble in water.
[22] The production method of any one of [17] - [21], wherein the anionic compound has a molecular weight of 30 - 5000.
[23] The production method of any one of [17] - [22], wherein the anionic compound is a bioactive substance.
[24] The production method of any one of [17] - [23], wherein the anionic compound is solid at 25°C.

### [Effect of the Invention]

According to the present invention, a method for controlling the skin permeation of an anionic compound is provided.

According to the present invention, moreover, a skin external preparation superior in safety and showing controlled skin permeability of the active ingredient is provided. For example, a skin external preparation showing promoted skin permeability is provided. In addition, for example, the active ingredient can be retained at a high concentration in the skin.

According to the present invention, moreover, a production method of a skin external preparation containing a ionic liquid is also provided.

### [Brief Description of the Drawings]

Fig. 1 is a graph showing the results of a skin permeation test of proline ethyl ester-ibuprofen salt and ibuprofen-PBS/EtOH solution.
Fig. 2 is a graph showing the results of a skin permeation test of proline ethyl ester-aspirin salt and aspirin-PBS/EtOH solution.
Fig. 3 is a graph showing the results of a skin permeation test of proline ethyl ester-indomethacin salt and indomethacin-PBS/EtOH solution.
Fig. 4 is a graph showing the results of a skin permeation test of alanine ethyl ester-ibuprofen salt and ibuprofen-PBS/EtOH solution.
Fig. 5 is a graph showing the results of a skin permeation test of alanine ethyl ester-aspirin salt and aspirin-PBS/EtOH solution.
Fig. 6 is a graph showing the results of a skin permeation test of phenylalanine ethyl ester-aspirin salt and aspirin-PBS/EtOH solution.
Fig. 7 is a graph showing the results of a skin permeation test of leucine ethyl ester-aspirin salt and aspirin-PBS/EtOH solution.
Fig. 8 is a graph showing the results of a skin permeation test of aspartic acid dimethyl ester-aspirin salt and aspirin-PBS/EtOH solution.
Fig. 9 is a graph showing the results of a skin permeation test of lysine methyl ester-diaspirin salt and aspirin-PBS/EtOH solution.
Fig. 10 is a graph showing the results of a skin permeation test of proline isopropyl ester-aspirin salt and aspirin-PBS/EtOH solution.
Fig. 11 is a graph showing the results of a skin permeation test of proline ethyl ester-ketoprofen salt and ketoprofen-PBS/EtOH solution.
Fig. 12 is a graph showing the results of a skin permeation test of proline ethyl ester-etodolac salt and etodolac-PBS/EtOH solution.
Fig. 13 is a graph showing the results of a skin permeation test of proline ethyl ester-naproxen salt and naproxen-PBS/EtOH solution.
Fig. 14 is a graph showing the results of a skin permeation test of proline ethyl ester-mycophenolic acid salt and mycophenolic acid-PBS/EtOH solution.
Fig. 15 is a graph showing the results of a skin permeation test of proline ethyl ester-loxoprofen salt and loxoprofen-PBS/EtOH solution.
Fig. 16 is a graph showing the results of a skin permeation test of proline ethyl ester-acetic acid salt and acetic acid-PBS/EtOH solution.
Fig. 17 is a graph showing the results of a skin permeation test of proline ethyl ester-penicillin salt and penicillin-PBS/EtOH solution.
Fig. 18 is a graph showing the results of a skin permeation test of proline ethyl ester-pantothenic acid salt and pantothenic acid-PBS/EtOH solution.
Fig. 19 is a graph showing the results of a skin permeation test of proline ethyl ester-glycyrrhetinic acid salt and glycyrrhetinic acid-PBS/EtOH solution. "GA" in the Figure shows glycyrrhetinic acid.
Fig. 20 is a graph showing the results of a skin permeation test of proline ethyl ester-adapalene salt and adapalene PBS/EtOH solution.
Fig. 21 is a graph showing the results of a cytotoxicity test.
Fig. 22 is a graph showing the results of an evaluation test of a medicament retention behavior in the skin.

### [Description of Embodiments]

### 1. Skin external preparation

The skin external preparation of the present invention is mainly characterized in that it contains an ionic liquid formed from an anionic compound and an amino acid ester.

In the present invention, the "ionic liquid" refers to a salt constituted of anion and cation, and capable of melting in a temperature region of not more than about 100°C.

The anionic compound and amino acid ester used for formation of an ionic liquid to be contained in the skin external preparation of the present invention are explained below.

### [Anionic compound]

The anionic compound to be used in the present invention is not particularly limited in the structure and the like as long as it can form an ionic liquid with the below-mentioned amino acid ester. In the present invention, the "anionic compound" means a compound capable of forming organic anion by electrolytic dissociation in water.

The anionic compound to be used in the present invention preferably has one or more (more preferably 1 - 100, particularly preferably 1 - 10) anionic functional groups. Examples of the functional group include carboxy group, sulfo group, phosphoric acid group, phosphono group and the like, with preference given to carboxy group.

The anionic compound to be used in the present invention may be one capable of being dissolved in solvents (water, glycerol, petrolatum, liquid paraffin etc.) generally used for dissolving a bioactive substance. The present invention is particularly preferably used for an anionic compound hardly soluble in these solvents, since it includes converting an anionic compound hardly soluble in these solvents into an ionic liquid (anionic compound itself is liquefacted). The hardly soluble anionic compound in the present invention can be defined by the solubility in water and, for example, the present invention can be preferably used for an anionic compound having solubility in water at 25°C of not more than 1 g/dl, further, not more than 0.5 g/dl, further, not more than 0.1 g/dl.

While the molecular weight of an anionic compound to be used in the present invention is not particularly limited, it is generally not more than 5000, preferably not more than 1000, more preferably not more than 750. Since skin permeability is more remarkably promoted, it is further preferably not more than 500, particularly preferably not more than 400. The molecular weight of the anionic compound is generally not less than 30, preferably not less than 40, more preferably not less than 50, further preferably not less than 100, particularly preferably not less than 150. A preferable range of the molecular weight of the anionic compound is generally 30 - 5000, preferably 40 - 1000, more preferably 50 - 500.

While an anionic compound to be used in the present invention preferably has physiological activity, the skin external preparation of the present invention may be one wherein the anionic compound does not have a physiological activity and amino acid constituting the below-mentioned amino acid ester has a physiological activity. In addition, both the anionic compound and the amino acid may have a physiological activity.

The state at 25°C of an anionic compound to be used in the present invention is not particularly limited, and may be any of solid, semisolid, liquid and the like. For example, to exhibit a skin permeability promoting effect, a solid anionic compound at 25°C can be preferably used.

Examples of the anionic compound to be used in the present invention include, but are not limited to, compounds having a carboxy group such as aspirin, mefenamic acid, indomethacin, diclofenac, etodolac, ibuprofen, ketoprofen, loxoprofen, naproxen, ketorolac, penicillin, ampicillin, levofloxacin, fexofenadine, levocetirizine, methotrexate, levodopa, 5-aminosalicylic acid (5-ASA), atorvastatin, captopril, valsartan, mycophenolic acid, adapalene, prostaglandin F2α, fenofibric acid, pyroglutamic acid, pantothenic acid, ferulic acid, docosahexaenoic acid, azelaic acid, glycyrrhetinic acid, acetic acid, penicillin and the like; compounds having a sulfo group such as taurine, azulene sulfonate and the like; compounds having a phosphoric acid group such as risedronic acid, 5-adenylic acid and the like; ascorbic acid and the like.

While an anionic compound to be used in the present invention may be an aromatic compound, it is not limited thereto. In the present invention, the "aromatic compound" refers to a compound having an aromatic ring and also encompasses aromatic hydrocarbon wherein aromatic ring is constituted of carbon atom alone and a hetero aromatic compound having hetero atom (e.g., oxygen atom, nitrogen atom etc.) on aromatic ring.

### [Amino acid ester]

Examples of the amino acid ester to be used in the present invention include amino acid alkyl ester, amino acid alkenyl ester, amino acid aralkyl ester and the like, with preference given to amino acid alkyl ester. The amino acid ester may be a monoester or diester, or other ester.

An amino acid ester to be used in the present invention is preferably a lower alcohol ester of amino acid. The lower alcohol constituting the lower alcohol ester may be any of linear chain and branched chain. The carbon number of the lower alcohol is preferably 1 - 6, more preferably 1 - 4. Specific examples of the lower alcohol include methanol, ethanol, 1-propanol, 2-propanol and the like, with preference given to ethanol.

As the amino acid constituting an amino acid ester to be used in the present invention, an organic compound having both amino group and carboxy group can be widely used, and proteinogenic amino acid is preferable. Specific examples of the amino acid include, but are not limited to, aliphatic amino acid (e.g., glycine, alanine), branched chain amino acid (e.g., isoleucine, leucine, valine), hydroxyl amino acid (e.g., serine, threonine), acidic amino acid (e.g., aspartic acid, glutamic acid), amide-type amino acid (e.g., asparagine, glutamine), basic amino acid (e.g., lysine, hydroxylysine, arginine, histidine), sulfur containing amino acid (e.g., cysteine, cystine, methionine), aromatic amino acid (e.g., phenylalanine, tyrosine), heterocyclic amino acid (e.g., tryptophan, histidine), imino acid (e.g., proline, 4-hydroxyproline) and the like. These amino acids may be any of D form, L form and DL form.

In the present invention, the properties (e.g., skin permeability, skin retentivity etc.) of an anionic compound converted into an ionic liquid vary depending on the kind of the amino acid constituting the amino acid ester, and various amino acids can be used according to the use and the like of the skin external preparation. Therefore, skin permeability of an anionic compound can be controlled by appropriately using various amino acids as the amino acid constituting an amino acid ester.

Specific examples of the amino acid ester to be used in the present invention include, but are not limited to, alanine ethyl ester, proline ethyl ester, proline isopropyl ester, leucine ethyl ester, phenylalanine ethyl ester, aspartic acid dimethyl ester, lysine methyl ester and the like.

A synthesis method of an amino acid ester to be used in the present invention is not particularly limited, and a method known per se can be used.

### [Synthesis method of ionic liquid (conversion of anionic compound into ionic liquid)]

A synthesis method of the ionic liquid is not particularly limited and a method known per se can be used. For example, an ionic liquid can be obtained by adding an anionic compound to a free form of an amino acid ester to 1/3-fold mole - 2-fold mole (e.g., equimolar) and appropriately agitating the mixture. Alternatively, a free form of an amino acid ester may be added to an anionic compound to 1/3-fold mole - 2-fold mole (e.g., equimolar) and then the mixture is appropriately stirred.

The content of an ionic liquid in the skin external preparation of the present invention can be appropriately controlled according to the kind of anion and cation constituting the ionic liquid, preparation form, the target to be applied with the skin external preparation and the like. It is generally 0.01 - 100 wt%, preferably 0.1 - 99 wt%, more preferably 0.5 - 50 wt%, relative to the whole preparation.

The skin external preparation of the present invention may further contain, in addition to the ionic liquid, additives generally used for formulation. Examples of the additive include excipient, suspending agent, dispersing agent, surfactant, thickener, antioxidant, preservative, pH adjuster, flavoring agent, colorant and the like.

The dosage form of the skin external preparation of the present invention is not particularly limited, and a dosage form known per se can be appropriately applied according to the use thereof and the like. Specific examples of the dosage form include, but are not limited to, embrocation such as ointment (aqueous ointment, oleagenous ointment etc.), cream, liquid, emulsion, gel, lotion, liniment, paste and the like; adhesive preparation such as cataplasm, plaster, tape, patch and the like; spray such as aerosol, spray and the like; suppository and the like.

A production method of the skin external preparation of the present invention is not particularly limited except that it includes formation of an ionic liquid from an anionic compound and an amino acid ester, and can be produced by appropriately combining conventionally-used methods according to the dosage form thereof and the like.

The use of the skin external preparation of the present invention is not particularly limited as long as it includes application of an active ingredient such as a bioactive substance and the like to the body by permeation through the skin. For example, the skin external preparation of the present invention includes pharmaceutical products for the skin, quasi-drugs for the skin, perfumery and cosmetics for the skin and the like. In the present invention, "skin" is a concept encompassing not only epidermis of the body (e.g., face, head, neck, breast, stomach, waist, back, hip, arm, leg, hand etc.) but also mucosa thereof.

Examples of the application target of the skin external preparation of the present invention include, but are not limited to, mammals (e.g., human, mouse, rat, hamster, rabbit, cat, dog, bovine, sheep, monkey etc.) and the like.

The dose and application frequency of the skin external preparation of the present invention to the target can be appropriately controlled according to the kind of anion and cation constituting the ionic liquid, preparation form, the target to be applied with the skin external preparation and the like.

### 2. Method for controlling skin permeation of anionic compound

In the present invention, as mentioned above, skin permeability of an anionic compound can be controlled by appropriately using various amino acids as the amino acid constituting an amino acid ester. Therefore, the present invention also provides a method for controlling the skin permeation of the anionic compound (hereinafter to be also referred to as "the controlling method of the present invention").

The controlling method of the present invention is mainly characterized by conversion of the anionic compound into an ionic liquid with an amino acid ester. The anionic compound and amino acid ester to be used for the preparation method of the present invention are similar to those explained for the skin external preparation of the present invention.

In the controlling method of the present invention, a method for converting an anionic compound into an ionic liquid with an amino acid ester is not particularly limited and a method known per se can be used. For example, an ionic liquid of an anionic compound can be obtained by adding the anionic compound to a free form of an amino acid ester to equimole and appropriately agitating the mixture. Alternatively, a free form of an amino acid ester may be added to an anionic compound to equimole and then the mixture is appropriately stirred.

The application target, dose and application frequency of the anionic compound converted into an ionic liquid with an amino acid ester are the same as those explained for the skin external preparation of the present invention.

In the present invention, "control" of the skin permeation of an anionic compound means at least one of promotion, maintenance and suppression of the skin permeation of the anionic compound and, for example, the controlling method of the present invention can be a method for promoting skin permeation of an anionic compound.

### [Examples]

The present invention is explained in more detail in the following by referring to Examples, which do not limit the present invention. The present invention may be modified without departing from the scope of the invention. The reagents, apparatuses and materials used in the present invention are commercially available unless particularly indicated.

### 1. Preparation method of test sample

The test samples used in the following skin permeation tests were each prepared by the following procedures.

### [Synthesis of amino acid ester free form]

### (Synthesis of proline ethyl ester free form)

Under room temperature conditions, proline (5 g) was dispersed in 50 mL of ethanol. Thereafter, 2.0-fold mole of thionyl chloride was added to proline, and the mixture was stirred for one day. Then, excess thionyl chloride and ethanol were removed by evaporation under reduced pressure to give proline ethyl ester hydrochloride. For the purpose to remove hydrochloric acid, water (10 mL), and then 2-fold mole of ammonia, were added to the obtained proline ethyl ester hydrochloride. Furthermore, 50 mL of ethyl acetate was added and the mixture was stirred at room temperature for about 2 hr. After stirring, the mixture was stood to allow for separation into 2 phases. The organic phase side was recovered, and excess ethyl acetate was removed by evaporation under reduced pressure to give a proline ethyl ester free form. The purity of the obtained substance was evaluated by NMR.

### (Synthesis of alanine ethyl ester free form)

Two-fold mole of ammonia was added to commercially available alanine ethyl ester hydrochloride (5 g, manufactured by Tokyo Chemical Industry Co., Ltd.), 50 mL of ethyl acetate was added and the mixture was stirred at room temperature for about 2 hr. After stirring, the mixture was stood to allow for separation into 2 phases. The organic phase side was recovered, and excess ethyl acetate was removed by evaporation under reduced pressure to give an alanine ethyl ester free form. The purity of the obtained substance was evaluated by NMR.

### (Synthesis of leucine ethyl ester free form)

Two-fold mole of ammonia was added to commercially available leucine ethyl ester hydrochloride (5 g, manufactured by Tokyo Chemical Industry Co., Ltd.), 50 mL of ethyl acetate was added and the mixture was stirred at room temperature for about 2 hr. After stirring, the mixture was stood to allow for separation into 2 phases. The organic phase side was recovered, and excess ethyl acetate was removed by evaporation under reduced pressure to give a leucine ethyl ester free form. The purity of the obtained substance was evaluated by NMR.

### (Synthesis of phenylalanine ethyl ester free form)

Two-fold mole of ammonia was added to commercially available phenylalanine ethyl ester hydrochloride (5 g, manufactured by Wako Pure Chemical Industries, Ltd.), 50 mL of ethyl acetate was added and the mixture was stirred at room temperature for about 2 hr. After stirring, the mixture was stood to allow for separation into 2 phases. The organic phase side was recovered, and excess ethyl acetate was removed by evaporation under reduced pressure to give a phenylalanine ethyl ester free form. The purity of the obtained substance was evaluated by NMR.

### (Synthesis of aspartic acid dimethyl ester free form)

Two-fold mole of ammonia was added to commercially available aspartic acid dimethyl ester hydrochloride (5 g, manufactured by Tokyo Chemical Industry Co., Ltd.), 50 mL of ethyl acetate was added and the mixture was stirred at room temperature for about 2 hr. After stirring, the mixture was stood to allow for separation into 2 phases. The organic phase side was recovered, and excess ethyl acetate was removed by evaporation under reduced pressure to give an aspartic acid dimethyl ester free form. The purity of the obtained substance was evaluated by NMR.

### (Synthesis of lysine methyl ester free form)

Two-fold mole of ammonia was added to commercially available lysine methyl ester hydrochloride (5 g, manufactured by KOKUSAN CHEMICAL Co., Ltd.), 50 mL of ethyl acetate was added and the mixture was stirred at room temperature for about 2 hr. After stirring, the mixture was stood to allow for separation into 2 phases. The organic phase side was recovered, and excess ethyl acetate was removed by evaporation under reduced pressure to give a lysine methyl ester free form. The purity of the obtained substance was evaluated by NMR.

### (Synthesis of proline isopropyl ester free form)

Under room temperature conditions, proline (5 g) was dispersed in 50 mL of isopropyl alcohol. Thereafter, 2.0-fold mole of thionyl chloride was added to proline, and the mixture was stirred for one day. Then, excess thionyl chloride and ethanol were removed by evaporation under reduced pressure to give proline isopropyl ester hydrochloride. For the purpose to remove hydrochloric acid, water (10 mL), and then 2-fold mole of ammonia, were added to the obtained proline isopropyl ester hydrochloride. Furthermore, 50 mL of ethyl acetate was added and the mixture was stirred at room temperature for about 2 hr. After stirring, the mixture was stood to allow for separation into 2 phases. The organic phase side was recovered, and excess ethyl acetate was removed by evaporation under reduced pressure to give a proline isopropyl ester free form. The purity of the obtained substance was evaluated by NMR.

### [Synthesis of ionic liquid]

### (Synthesis of proline ethyl ester-ibuprofen salt)

Ibuprofen powder (manufactured by Wako Pure Chemical Industries, Ltd.) was added to proline ethyl ester free form tobe equimolar, and the mixture was stirred at 40°C for 2 hr to give a proline ethyl ester-ibuprofen salt (oily substance).

### (Synthesis of proline ethyl ester-aspirin salt)

Aspirin (manufactured by Wako Pure Chemical Industries, Ltd.) was added to proline ethyl ester free form to be equimolar, and the mixture was stirred at room temperature for 8 hr to give a proline ethyl ester-aspirin salt (oily substance).

### (Synthesis of proline ethyl ester-indomethacin salt)

Indomethacin (manufactured by Nacalai Tesque) was added to proline ethyl ester free form to be equimolar, and the mixture was stirred at room temperature for 8 hr to give a proline ethyl ester-indomethacin salt (oily substance).

### (Synthesis of alanine ethyl ester-aspirin salt)

Aspirin (manufactured by Wako Pure Chemical Industries, Ltd.) was added to alanine ethyl ester free form to be equimolar, and the mixture was stirred at room temperature for 8 hr to give an alanine ethyl ester-aspirin salt (oily substance).

### (Synthesis of alanine ethyl ester-ibuprofen salt)

Ibuprofen (manufactured by Wako Pure Chemical Industries, Ltd.) was added to alanine ethyl ester free form to be equimolar, and the mixture was stirred at room temperature for 8 hr to give an alanine ethyl ester-ibuprofen salt (oily substance).

### (Synthesis of leucine ethyl ester-aspirin salt)

Aspirin (manufactured by Wako Pure Chemical Industries, Ltd.) was added to leucine ethyl ester free form to be equimolar, and the mixture was stirred at room temperature for 8 hr to give a leucine ethyl ester-aspirin salt (oily substance).

### (Synthesis of phenylalanine ethyl ester-aspirin salt)

Aspirin (manufactured by Wako Pure Chemical Industries, Ltd.) was added to phenylalanine ethyl ester free form to be equimolar, and the mixture was stirred at room temperature for 8 hr to give a phenylalanine ethyl ester-aspirin salt (oily substance).

### (Synthesis of aspartic acid dimethyl ester-aspirin salt)

Aspirin (manufactured by Wako Pure Chemical Industries, Ltd.) was added to aspartic acid dimethyl ester free form to be equimolar, and the mixture was stirred at room temperature for 8 hr to give an aspartic acid dimethyl ester-aspirin salt (oily substance).

### (Synthesis of lysine methyl ester-diaspirin salt)

Aspirin (manufactured by Wako Pure Chemical Industries, Ltd.) was added to lysine methyl ester free form to be 2-fold mole, and the mixture was stirred at room temperature for 8 hr to give a lysine methyl ester-diaspirin salt (oily substance).

### (Synthesis of proline isopropyl ester-aspirin salt)

Aspirin (manufactured by Wako Pure Chemical Industries, Ltd.) was added to proline isopropyl ester free form to be a 1/3-fold molar, and the mixture was stirred at room temperature for 8 hr to give a proline isopropyl ester-aspirin salt (oily substance).

### (Synthesis of proline ethyl ester-ketoprofen salt)

Ketoprofen (manufactured by Tokyo Chemical Industry Co., Ltd.) was added to proline ethyl ester free form to be equimolar, and the mixture was stirred at room temperature for 8 hr to give a proline ethyl ester-ketoprofen salt (oily substance).

### (Synthesis of proline ethyl ester-etodolac salt)

Etodolac (manufactured by Tokyo Chemical Industry Co., Ltd.) was added to proline ethyl ester free form to be a 1/2-fold molar, and the mixture was stirred at room temperature for 8 hr to give a proline ethyl ester-etodolac salt (oily substance).

### (Synthesis of proline ethyl ester-naproxen salt)

Naproxen (manufactured by Tokyo Chemical Industry Co., Ltd.) was added to proline ethyl ester free form to be a 1/2-fold molar, and the mixture was stirred at room temperature for 8 hr to give a proline ethyl ester-naproxen salt (oily substance).

### (Synthesis of proline ethyl ester-mycophenolic acid salt)

Mycophenolic acid (manufactured by Tokyo Chemical Industry Co., Ltd.) was added to proline ethyl ester free form to be equimolar, and the mixture was stirred at room temperature for 8 hr to give a proline ethyl ester-mycophenol salt (oily substance).

### (Synthesis of proline ethyl ester-loxoprofen salt)

Loxoprofen (manufactured by Tokyo Chemical Industry Co., Ltd.) was added to proline ethyl ester free form to be equimolar, and the mixture was stirred at room temperature for 8 hr to give a proline ethyl ester-loxoprofen salt (oily substance).

### (Synthesis of proline ethyl ester-acetic acid salt)

Acetic acid (manufactured by Tokyo Chemical Industry Co., Ltd.) was added to proline ethyl ester free form to be equimolar, and the mixture was stirred at room temperature for 8 hr to give a proline ethyl ester-acetic acid salt (oily substance).

### (Synthesis of proline ethyl ester-penicillin salt)

Penicillin (manufactured by Tokyo Chemical Industry Co., Ltd.) was added to proline ethyl ester free form to be equimolar, and the mixture was stirred at room temperature for 8 hr to give a proline ethyl ester-penicillin salt (oily substance). Since the solution became highly viscous, ethanol was added to 50% of the total weight and the mixture was subjected to a skin permeation test.

### (Synthesis of proline ethyl ester-pantothenic acid salt)

Pantothenic acid (manufactured by Tokyo Chemical Industry Co., Ltd.) was added to proline ethyl ester free form to be equimolar, and the mixture was stirred at room temperature for 8 hr to give a proline ethyl ester-pantothenic acid salt (oily substance).

### (Synthesis of proline ethyl ester-glycyrrhetinic acid salt)

Glycyrrhetinic acid (manufactured by Tokyo Chemical Industry Co., Ltd.) was added to proline ethyl ester free form to be equimolar, and the mixture was stirred at room temperature for 8 hr to give a proline ethyl ester-glycyrrhetinic acid salt (oily substance). Since the solution became highly viscous, ethanol was added to 50% of the total weight and the mixture was subjected to a skin permeation test.

### (Synthesis of proline ethyl ester-adapalene salt)

Adapalene (manufactured by Tokyo Chemical Industry Co., Ltd.) was added to proline ethyl ester free form to be equimolar, and the mixture was stirred at room temperature for 8 hr to give a proline ethyl ester-adapalene salt (oily substance). Since the solution became highly viscous, ethanol was added to 50% of the total weight and the mixture was subjected to a skin permeation test.

### [Preparation of drug solution]

### (Preparation of ibuprofen-PBS/EtOH solution)

Sodium chloride, potassium chloride, disodium hydrogen phosphate dodecahydrate, and potassium dihydrogen phosphate were each dissolved in ion exchange water at 8 g/L, 0.2 g/L, 2.9 g/L, and 0.2 g/L, respectively. Thereafter, the mixture was adjusted to pH 7.4 with hydrochloric acid or sodium hydroxide. The thus-prepared phosphate buffer (PBS) was mixed with ethanol (EtOH) at 1:1 (volume ratio) to give a PBS/EtOH mixture. Ibuprofen was added to the PBS/EtOH mixture to saturation and the mixture was stirred at room temperature for 3 hr. Thereafter, excess ibuprofen remaining undissolved was removed by filtration to give an ibuprofen-PBS/EtOH solution.

### (Preparation of aspirin-PBS/EtOH solution)

Aspirin was added to the PBS/EtOH mixture produced as mentioned above to saturation and the mixture was stirred at room temperature for 3 hr. Thereafter, excess aspirin remaining undissolved was removed by filtration to give an aspirin-PBS/EtOH solution.

### (Preparation of indomethacin-PBS/EtOH solution)

Indomethacin was added to the PBS/EtOH mixture produced as mentioned above to saturation and the mixture was stirred at room temperature for 3 hr. Thereafter, excess indomethacin remaining undissolved was removed by filtration to give an indomethacin-PBS/EtOH solution.

### (Preparation of ketoprofen-PBS/EtOH solution)

Ketoprofen was added to the PBS/EtOH mixture produced as mentioned above to saturation and the mixture was stirred at room temperature for 3 hr. Thereafter, excess ketoprofen remaining undissolved was removed by filtration to give a ketoprofen-PBS/EtOH solution.

### (Preparation of etodolac-PBS/EtOH solution)

Etodolac was added to the PBS/EtOH mixture produced as mentioned above to saturation and the mixture was stirred at room temperature for 3 hr. Thereafter, excess etodolac remaining undissolved was removed by filtration to give an etodolac-PBS/EtOH solution.

### (Preparation of naproxen-PBS/EtOH solution)

Naproxen was added to the PBS/EtOH mixture produced as mentioned above to saturation and the mixture was stirred at room temperature for 3 hr. Thereafter, excess naproxen remaining undissolved was removed by filtration to give a naproxen-PBS/EtOH solution.

### (Preparation of mycophenolic acid-PBS/EtOH solution)

Mycophenolic acid was added to the PBS/EtOH mixture produced as mentioned above to saturation and the mixture was stirred at room temperature for 3 hr. Thereafter, excess mycophenolic acid remaining undissolved was removed by filtration to give a mycophenolic acid-PBS/EtOH solution.

### (Preparation of loxoprofen-PBS/EtOH solution)

Loxoprofen was added to the PBS/EtOH mixture produced as mentioned above to the same weight concentration as the proline ethyl ester-loxoprofen salt and the mixture was stirred at room temperature for 3 hr. In this way, a mycophenolic acid-PBS/EtOH solution was prepared.

### (Preparation of acetic acid-PBS/EtOH solution)

Acetic acid was added to the PBS/EtOH mixture produced as mentioned above to saturation and the mixture was stirred at room temperature for 3 hr. Thereafter, excess acetic acid remaining undissolved was removed by filtration to give an acetic acid-PBS/EtOH solution.

### (Preparation of penicillin-PBS/EtOH solution)

Penicillin was added to the PBS/EtOH mixture produced as mentioned above to saturation and the mixture was stirred at room temperature for 3 hr. Thereafter, excess penicillin remaining undissolved was removed by filtration to give a penicillin-PBS/EtOH solution.

### (Preparation of pantothenic acid-PBS/EtOH solution)

Pantothenic acid was added to the PBS/EtOH mixture produced as mentioned above to saturation and the mixture was stirred at room temperature for 3 hr. Thereafter, excess pantothenic acid remaining undissolved was removed by filtration to give a pantothenic acid-PBS/EtOH solution.

### (Preparation of glycyrrhetinic acid-PBS/EtOH solution)

Glycyrrhetinic acid was added to the PBS/EtOH mixture produced as mentioned above to saturation and the mixture was stirred at room temperature for 3 hr. Thereafter, excess glycyrrhetinic acid remaining undissolved was removed by filtration to give a glycyrrhetinic acid-PBS/EtOH solution.

### (Preparation of adapalene-PBS/EtOH solution)

Adapalene was added to the PBS/EtOH mixture produced as mentioned above to saturation and the mixture was stirred at room temperature for 3 hr. Thereafter, excess adapalene remaining undissolved was removed by filtration to give an adapalene-PBS/EtOH solution.

### 2. Drug analysis method

In the following skin permeation test, each drug was analyzed by the following procedures.

### (Aspirin analysis method)

Buffer composition: 10 mM KH₂PO₄ aqueous solution was prepared and adjusted to pH 3.5 with phosphoric acid. This aqueous solution and methanol were mixed at a volume ratio of 7:3, and the mixture was deaerated and analyzed.

As an analysis column, ODS-3 column (4.6 mm×250 mm) manufactured by GL Sciences Inc. was used. The flow rate was set to 0.8 mL/min, and the column temperature was set to 40°C, and aspirin was analyzed from the absorption wavelength at 260 nm.

### (Indomethacin analysis method)

Buffer composition: 0.1% phosphoric acid solution was prepared. This phosphoric acid liquid and methanol were mixed at a volume ratio of 1:3, and the mixture was deaerated and analyzed.

As an analysis column, ODS-3 column (4.6 mmx250 mm) manufactured by GL Sciences Inc. was used. The flow rate was set to 0.8 mL/min, and the column temperature was set to 40°C, and indomethacin was analyzed from the absorption wavelength at 254 nm.

### (Ibuprofen analysis method)

Buffer composition: 20 mM phosphoric acid solution was prepared. This phosphoric acid liquid and acetonitrile were mixed at a volume ratio of 55:45, and the mixture was deaerated and analyzed.

As an analysis column, L-column ODS (4.6 mm×150 mm) manufactured by Chemicals Evaluation and Research Institute, Japan was used. The flow rate was set to 1.0 mL/min, and the column temperature was set to 40°C, and ibuprofen was analyzed from the absorption wavelength at 230 nm.

### (Analysis methods of ketoprofen, etodolac, naproxen, mycophenolic acid, loxoprofen, acetic acid, penicillin, pantothenic acid, glycyrrhetinic acid and adapalene)

Ketoprofen, etodolac, naproxen, mycophenolic acid, loxoprofen, acetic acid, penicillin, pantothenic acid, glycyrrhetinic acid and adapalene were each analyzed by a method similar to the analysis method of ibuprofen.

### 3. Skin permeation test

Using the above-mentioned test samples, a skin permeation test was performed by the following procedures.

### [Skin permeation test]

A fat layer was removed from the skin of Yucatan pig, and the skin was mounted on a franz type diffusion cell with the corium side thereof being the receptor bath side, such that the bath solution contacted the corium side through a circular opening with diameter 15 mm. Then, a silicon rubber sheet having a 15 mm circular opening was adhered to the stratum corneum layer side of the skin, and 0.3 mL of a test sample (ionic liquid or drug solution) was applied to the stratum corneum layer side in the opening surface of the silicon rubber sheet. Thereafter, 12 mL of a mixed solution of phosphate buffered saline and ethanol was filled in a receptor bath, and the mixture was stood at 37°C while agitating with a magnetic bar. The solution was collected from the receptor bath by 1 mL at regular time intervals up to 96 hr, and the concentration of the anionic compound in the solution was measured. Every time 1 mL of the solution was collected from the receptor bath, 1 mL of a fresh mixed solution of phosphate buffered saline and ethanol was added to the receptor bath.
product name: Yucatan Micropig Skin set
   female 5 months of age, about 20 kg
   isolated skin about 10 cm×10 cm per sheet
   16 sheets from left and right of one pig (shoulder 2, back 6, dorsal part 6, buttock 2)

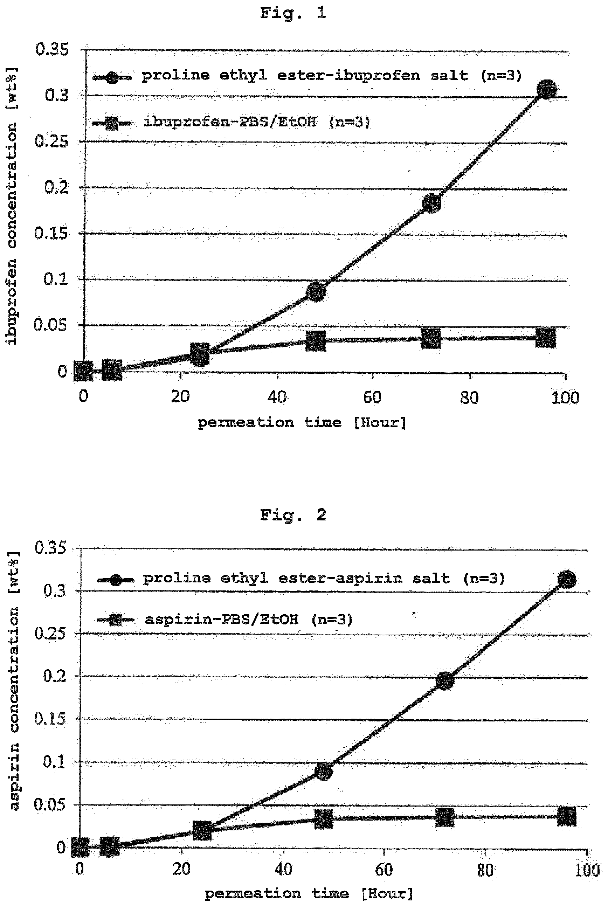

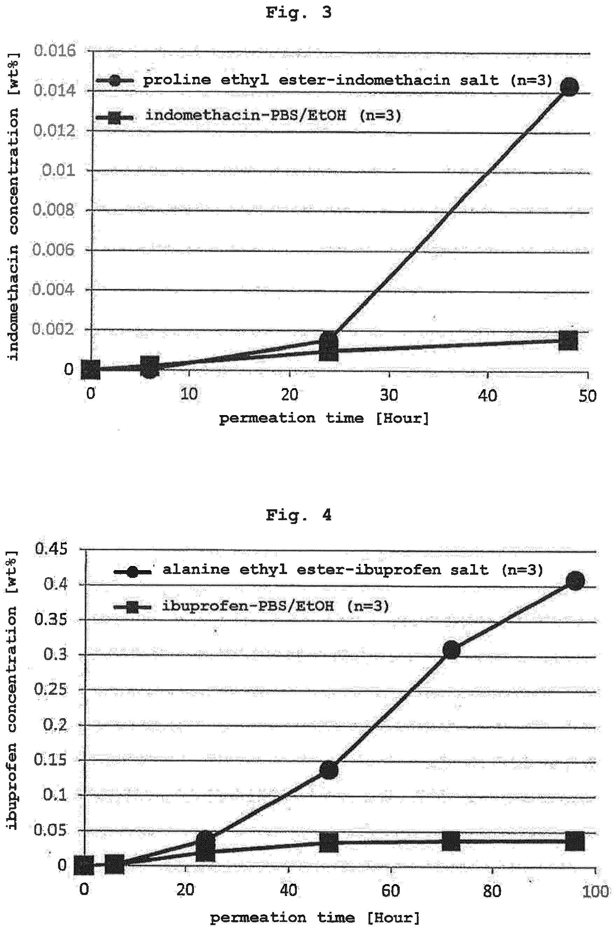

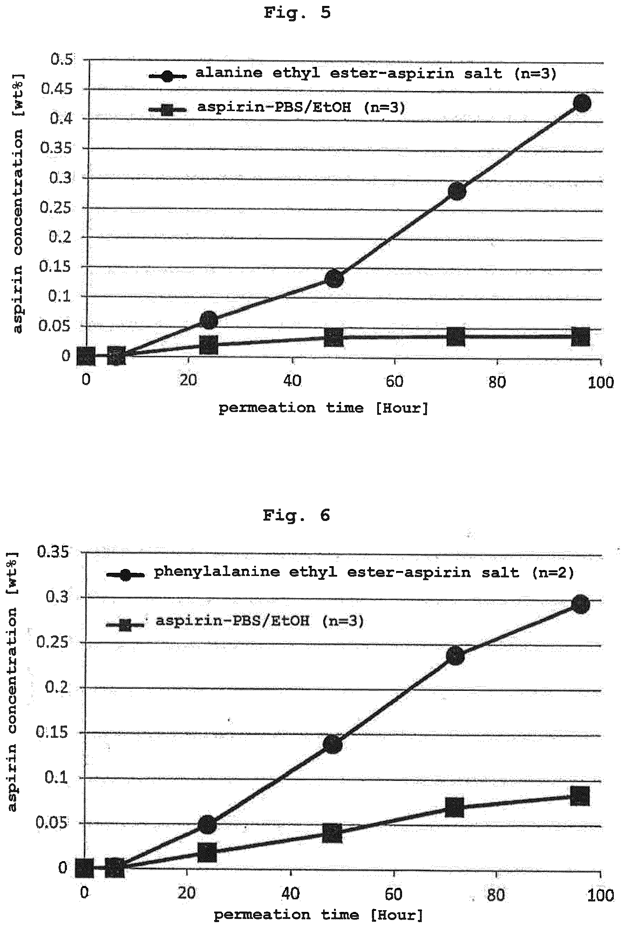

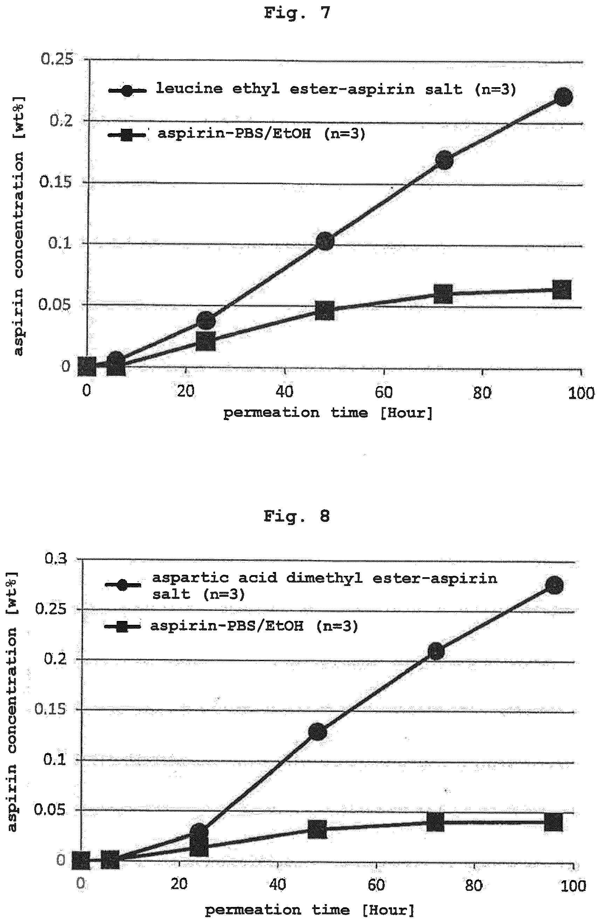

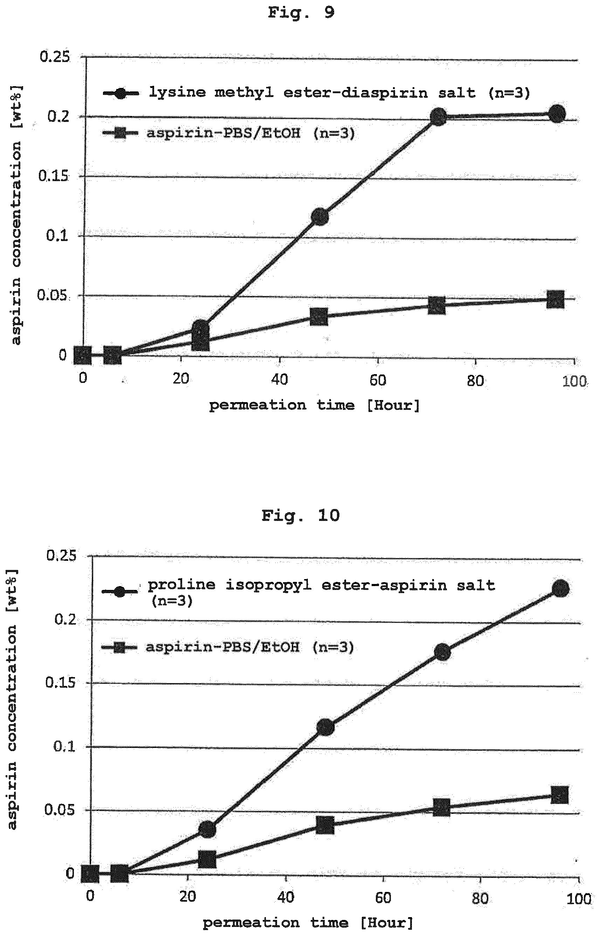

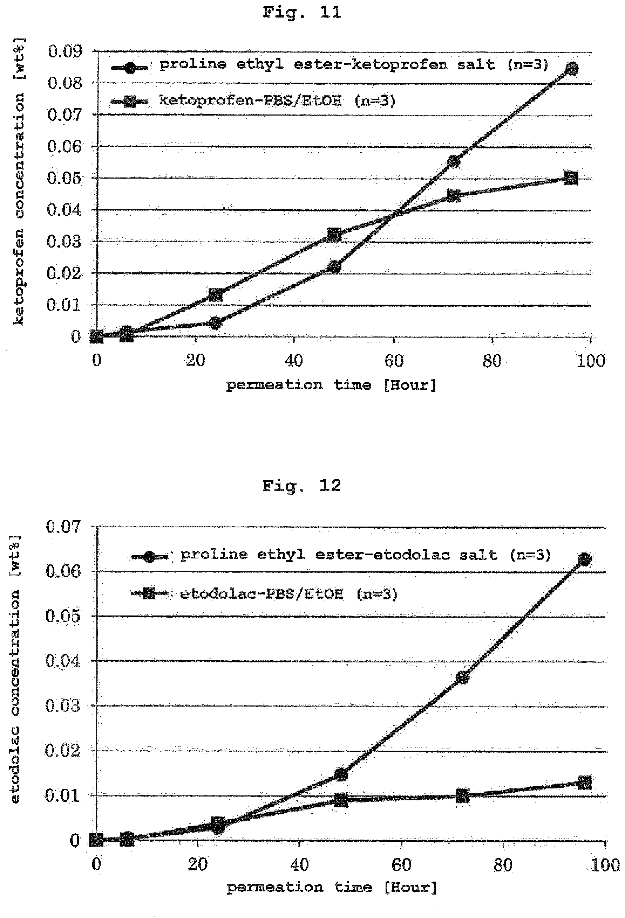

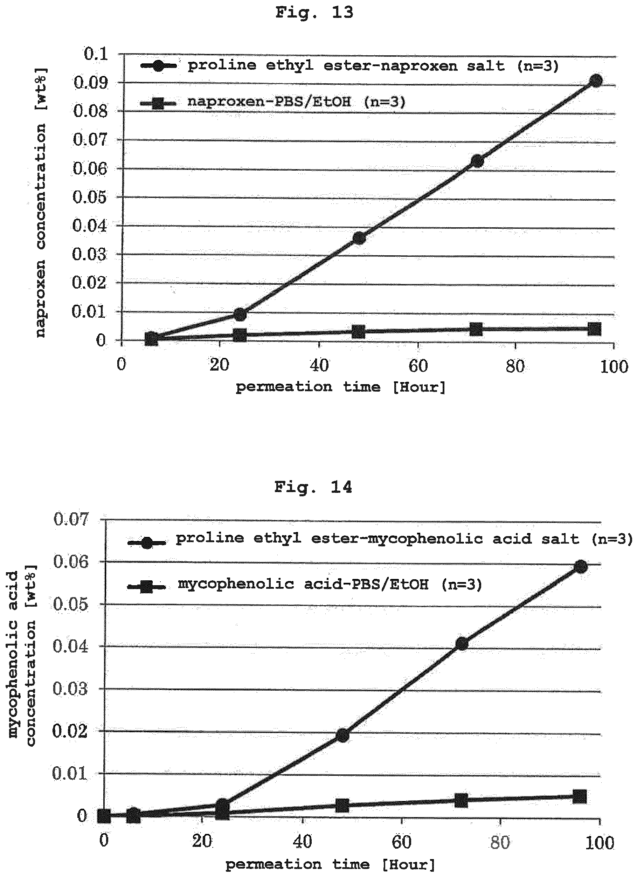

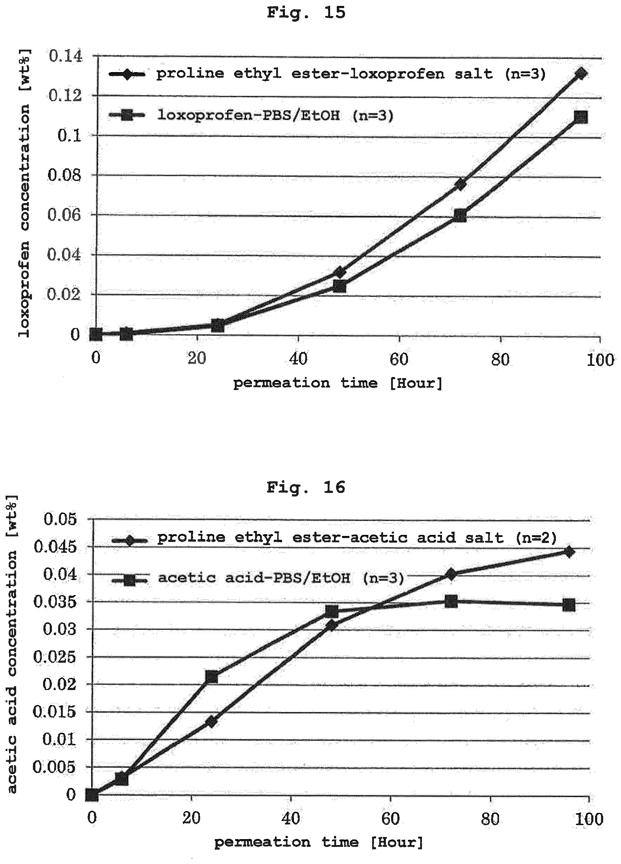

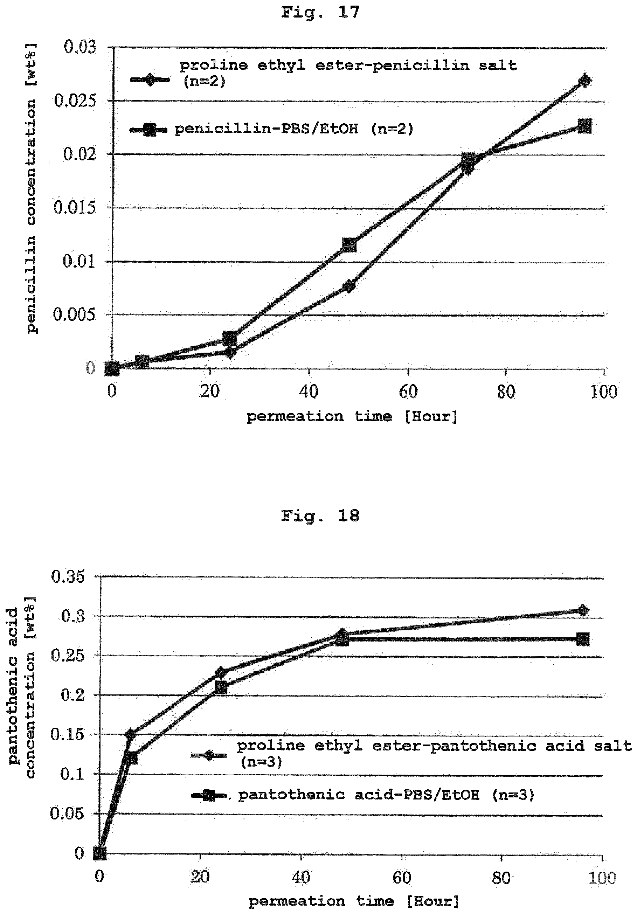

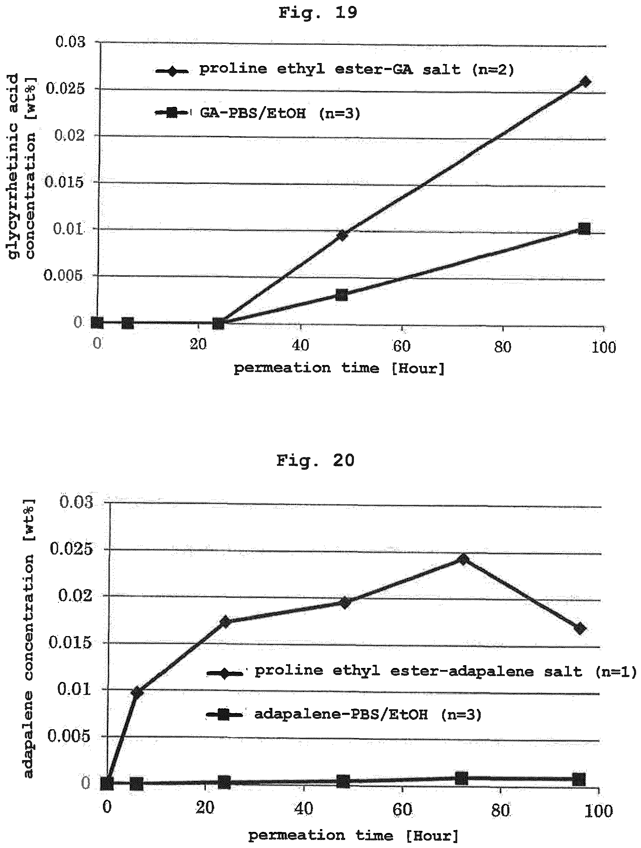

The results of a skin permeation test of proline ethyl ester-ibuprofen salt (n=3) and ibuprofen-PBS/EtOH solution (n=3) are shown in Fig. 1, the results of a skin permeation test of proline ethyl ester-aspirin salt (n=3) and aspirin-PBS/EtOH solution (n=3) are shown in Fig. 2, the results of a skin permeation test of proline ethyl ester-indomethacin salt (n=3) and indomethacin-PBS/EtOH solution (n=3) are shown in Fig. 3, the results of a skin permeation test of alanine ethyl ester-ibuprofen salt (n=3) and ibuprofen-PBS/EtOH solution (n=3) are shown in Fig. 4, the results of a skin permeation test of alanine ethyl ester-aspirin salt (n=3) and aspirin-PBS/EtOH solution (n=3) are shown in Fig. 5, the results of a skin permeation test of phenylalanine ethyl ester-aspirin salt (n=2) and aspirin-PBS/EtOH solution (n=3) are shown in Fig. 6, the results of a skin permeation test of leucine ethyl ester-aspirin salt (n=3) and aspirin-PBS/EtOH solution (n=3) are shown in Fig. 7, the results of a skin permeation test of aspartic acid dimethyl ester-aspirin salt (n=3) and aspirin-PBS/EtOH solution (n=3) are shown in Fig. 8, the results of a skin permeation test of lysine methyl ester-diaspirin salt (n=3) and aspirin-PBS/EtOH solution (n=3) are shown in Fig. 9, the results of a skin permeation test of proline isopropyl ester-aspirin salt (n=3) and aspirin-PBS/EtOH solution (n=3) are shown in Fig. 10, the results of a skin permeation test of proline ethyl ester-ketoprofen salt (n=3) and ketoprofen-PBS/EtOH solution (n=3) are shown in Fig. 11, the results of a skin permeation test of proline ethyl ester-etodolac salt (n=3) and etodolac-PBS/EtOH solution (n=3) are shown in Fig. 12, the results of a skin permeation test of proline ethyl ester-naproxen salt (n=3) and naproxen-PBS/EtOH solution (n=3) are shown in Fig. 13, the results of a skin permeation test of proline ethyl ester-mycophenolic acid salt (n=3) and mycophenolic acid-PBS/EtOH solution (n=3) are shown in Fig. 14, the results of a skin permeation test of proline ethyl ester-loxoprofen salt (n=3) and loxoprofen-PBS/EtOH solution (n=3) are shown in Fig. 15, the results of a skin permeation test of proline ethyl ester-acetic acid salt (n=2) and acetic acid-PBS/EtOH solution (n=3) are shown in Fig. 16, the results of a skin permeation test of proline ethyl ester-penicillin salt (n=2) and penicillin-PBS/EtOH solution (n=2) are shown in Fig. 17, the results of a skin permeation test of proline ethyl ester-pantothenic acid salt (n=3) and pantothenic acid-PBS/EtOH solution (n=3) are shown in Fig. 18, the results of a skin permeation test of proline ethyl ester-glycyrrhetinic acid salt (n=2) and glycyrrhetinic acid-PBS/EtOH solution (n=3) are shown in Fig. 19, and the results of a skin permeation test of proline ethyl ester-adapalene salt (n=1) and adapalene PBS/EtOH solution (n=3) are shown in Fig. 20.

As is clear from the results of Figs. 1 - 20, all ionic liquids showed very high skin permeation rates as compared to the drug solutions.

### 4. Cytotoxicity test

Using the above-mentioned proline ethyl esters, a cytotoxicity test was performed by the following procedures.

### [Cytotoxicity test]

Proline ethyl ester was dissolved in Krebs Ringer Hepes buffer at 200 mM, and the obtained solution (pH 7.4) was mixed with Dulbecco's modified Eagle culture medium at a volume ratio of 1:3. The solution was appropriately diluted stepwisely within the range of 0.01 - 50 mM with a solution of Krebs Ringer Hepes buffer and Dulbecco's modified Eagle culture medium mixed at a volume ratio of 1:3, and added by 200 µL to each well of a 96 well culture dish seeded with mouse fibroblast 10T1/2 cells at 5×10³ cells/well 15 hours before. The cells were exposed to the solution in a CO₂ incubator for 20 hr, and the solution was collected from each well and the cells were washed with Dulbecco's modified Eagle culture medium. Then, a culture medium containing a cell counting kit-8 reagent was added to each well by 150 µL, and the mixture was stood in a CO₂ incubator for 2 hr, and the absorbance of the solution was measured at a wavelength of 450 nm. As a control, a solution of Krebs Ringer Hepes buffer and Dulbecco's modified Eagle culture medium free of proline ethyl ester mixed at a volume ratio of 1:3 was prepared, mouse fibroblast 10T1/2 cells were immersed in the solution for 15 hr, and the absorbance was measured. The specific survival rate of proline ethyl ester was calculated with the absorbance of the control as 1.

In addition, using proline, ethanol, proline-tetrabutylphosphonium salt, prolinol and triethanolamine proline ethyl ester, a cytotoxicity test was performed. These cytotoxicity tests were performed in the same manner as above except that proline, proline-tetrabutylphosphonium salt and triethanolamine were used instead of proline ethyl ester. The proline-tetrabutylphosphonium salt was synthesized by the following procedures.

### (Synthesis of proline-tetrabutylphosphonium salt)

Commercially available 40% aqueous tetrabutylphosphonium hydroxide solution (manufactured by HOKKO CHEMICAL INDUSTRY CO., LTD.) (30 g) was added to proline (5 g) to be equimolar. The mixture was concentrated by evaporation and water in the mixture was evaporated. A proline-tetrabutylphosphonium salt was finally obtained (colorless transparent oily substance).

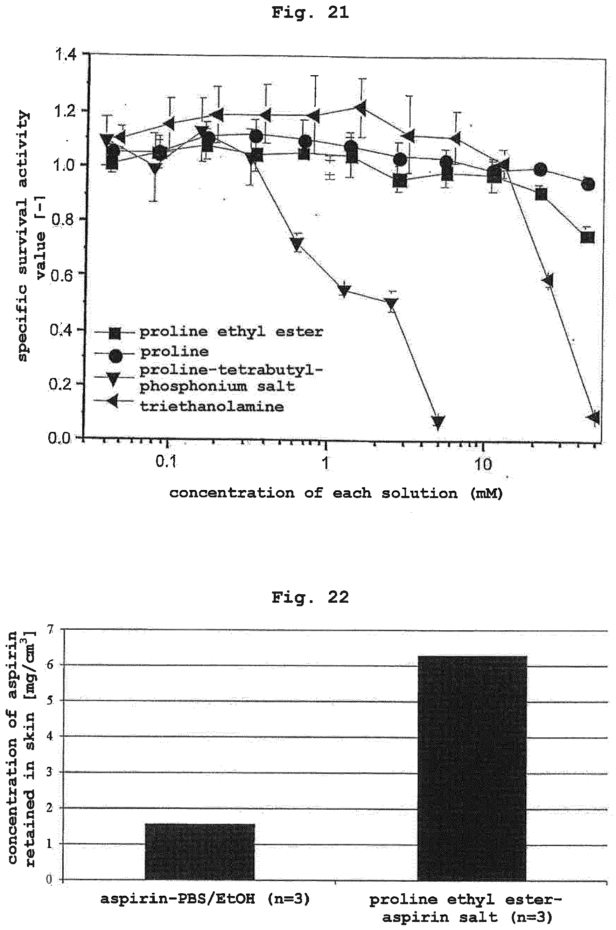

The results are shown in Fig. 21.

As is clear from the results of Fig. 21, it was confirmed that proline ethyl ester shows low cytotoxicity as compared to triethanolamine generally used for perfumery, cosmetics and the like, and is superior in safety.

In contrast, the proline-tetrabutylphosphonium salt which is an ionic liquid containing tetrabutylphosphonium as a constituent cation showed very high cytotoxicity.

### 5. Evaluation test of medicament retention behavior in skin

Using the above-mentioned proline ethyl ester-aspirin salt and aspirin-PBS/EtOH solution, an evaluation test of medicament retention behavior in the skin was performed by the following procedures.

### [Evaluation test of medicament retention behavior in skin]

By a method similar to the above-mentioned skin permeation test, 0.3 mL of a test sample (proline ethyl ester-aspirin salt or aspirin-PBS/EtOH solution) was applied to the stratum corneum layer side of the skin of Yucatan pig. Thereafter, 12 mL of a mixed solution of phosphate buffered saline and ethanol was filled in a receptor bath, and the mixture was stood at 37°C for 72 hr while agitating with a magnetic bar. Thereafter, the skin applied with each test sample was recovered, the surface was washed well with water, immersed in 5 mL of ethyl acetate, and aspirin retained in the skin was extracted with stirring by a magnetic stirrer at 350 rpm at room temperature for 5 hr. The thus-obtained ethyl acetate extraction solution was analyzed by HPLC, and the concentration of aspirin retained in the skin by the application of each test sample was calculated.

The results are shown in Fig. 22.

As is clear from the results of Fig. 22, it was shown that aspirin can be retained in the skin at a markedly high concentration by converting same into an ionic liquid.

### [Industrial Applicability]

According to the present invention, a skin external preparation capable of controlling the skin permeability of the active ingredient, and superior in safety can be provided.

This application is based on a patent application No. 2015-13329 filed in Japan (filing date: January 27, 2015), the contents of which are incorporated in full herein.

## Claims

1. A method for controlling skin permeation of an anionic compound, comprising converting the anionic compound into an ionic liquid with an amino acid ester.

2. The method for controlling the skin permeation according to claim 1, wherein the amino acid ester is a lower alcohol ester of amino acid.

3. The method for controlling the skin permeation according to claim 2, wherein the lower alcohol is at least one kind selected from the group consisting of methanol, ethanol, 1-propanol and 2-propanol.

4. The method for controlling the skin permeation according to any one of claims 1 to 3, wherein the anionic compound has at least one kind of functional group selected from the group consisting of a carboxy group, a sulfo group, a phosphoric acid group and a phosphono group.

5. The method for controlling the skin permeation according to any one of claims 1 to 4, wherein the anionic compound is hardly soluble in water.

6. The method for controlling the skin permeation according to any one of claims 1 to 5, wherein the anionic compound has a molecular weight of 30 - 5000.

7. The method for controlling the skin permeation according to any one of claims 1 to 6, wherein the anionic compound is a bioactive substance.

8. The method for controlling the skin permeation according to any one of claims 1 to 7, wherein the anionic compound is solid at 25°C.

9. A skin external preparation comprising an ionic liquid formed by an anionic compound and an amino acid ester.

10. The skin external preparation according to claim 9, wherein the amino acid ester is a lower alcohol ester of amino acid.

11. The skin external preparation according to claim 10, wherein the lower alcohol is at least one kind selected from the group consisting of methanol, ethanol, 1-propanol and 2-propanol.

12. The skin external preparation according to any one of claims 9 to 11, wherein the anionic compound has at least one kind of functional group selected from the group consisting of a carboxy group, a sulfo group, a phosphoric acid group and a phosphono group.

13. The skin external preparation according to any one of claims 9 to 12, wherein the anionic compound is hardly soluble in water.

14. The skin external preparation according to any one of claims 9 to 13, wherein the anionic compound has a molecular weight of 30 - 5000.

15. The skin external preparation according to any one of claims 9 to 14, wherein the anionic compound is a bioactive substance.

16. The skin external preparation according to any one of claims 9 to 15, wherein the anionic compound is solid at 25°C.

17. A method of producing a skin external preparation comprising an ionic liquid, comprising forming the ionic liquid from an anionic compound and an amino acid ester.

18. The production method according to claim 17, wherein the amino acid ester is a lower alcohol ester of amino acid.

19. The production method according to claim 18, wherein the lower alcohol is at least one kind selected from the group consisting of methanol, ethanol, 1-propanol and 2-propanol.

20. The production method according to any one of claims 17 to 19, wherein the anionic compound has at least one kind of functional group selected from the group consisting of a carboxy group, a sulfo group, a phosphoric acid group and a phosphono group.

21. The production method according to any one of claims 17 to 20, wherein the anionic compound is hardly soluble in water.

22. The production method according to any one of claims 17 to 21, wherein the anionic compound has a molecular weight of 30 - 5000.

23. The production method according to any one of claims 17 to 22, wherein the anionic compound is a bioactive substance.

24. The production method according to any one of claims 17 to 23, wherein the anionic compound is solid at 25°C.
